# EUROPEAN PATENT APPLICATION

(11) **EP 4 099 333 A2**
(43) Date of publication of application: **07.12.2022**
(21) Application number: 22191040.9
(22) Date of filing: 18.08.2022
(51) Int. Cl.: G16C 20/30

(54) **METHOD AND APPARATUS FOR TRAINING COMPOUND PROPERTY PEDICTION MODEL, STORAGE MEDIUM AND COMPUTER PROGRAM PRODUCT**

(30) Priority: 07.12.2021 CN 202111482319
(71) Applicant: Beijing Baidu Netcom Science Technology Co., Ltd., Beijing 100085 (CN)
(72) Inventor: ZHANG, Shanzhuo, Beijing, 100085 (CN); LIU, Lihang, Beijing, 100085 (CN); HUANG, Yueyang, Beijing, 100085 (CN); HE, Donglong, Beijing, 100085 (CN); FANG, Xiaomin, Beijing, 100085 (CN); ZHANG, Xiaonan, Beijing, 100085 (CN); WANG, Fan, Beijing, 100085 (CN); HE, Jingzhou, Beijing, 100085 (CN)
(74) Representative: Lucke, Andreas

(57) **Abstract**

A method and apparatus for training a compound property prediction model, a storage medium and a program product. An implementation of the method comprises: acquiring an unannotated compound data set; pre-training a graph neural network using the unannotated compound data set to obtain a pre-trained graph neural network; acquiring a plurality of annotated compound data sets, each annotated compound data set being annotated with one kind of compound property; and performing multi-task training on the pre-trained graph neural network using the plurality of annotated compound data sets, to obtain a compound property prediction model, the compound property prediction model being used to predict a plurality kinds of properties of a compound.

## Description

### TECHNICAL FIELD

The present disclosure relates to the field of artificial intelligence technology, and particularly to the field of deep learning technology.

### BACKGROUND

Based on long-term research, development and practice on drugs, researchers have found that the performance of a candidate compound in late-stage clinical trials is largely related to the absorption, distribution, metabolism, excretion and toxicity of the compounds in human bodies. Therefore, the accurate prediction for the ADMET properties of candidate compounds can help to quickly screen compounds with a higher potential success rate in the early stage of the research and development of the drugs, thereby shortening the research and development cycle of a new drug and reducing research costs.

The core of an ADMET prediction platform is an algorithm model, and the existing ADMET prediction platform usually applies a certain deep learning model. However, the deep learning model needs to acquire a large amount of annotated training data for supervised training. If the training data is insufficient, the improvement for the effect of the model will be limited.

### SUMMARY

Embodiments of the present disclosure propose a method and apparatus for training a compound property prediction model, a storage medium and a program product.

In a first aspect, some embodiments of the present disclosure provide a method for training a compound property prediction model. The method includes: acquiring an unannotated compound data set; pre-training a graph neural network using the unannotated compound data set, to obtain a pre-trained graph neural network; acquiring a plurality of annotated compound data sets, each annotated compound data set being annotated with one kind of compound property; and performing multi-task training on the pre-trained graph neural network using the plurality of annotated compound data sets, to obtain a compound property prediction model, the compound property prediction model being used to predict a plurality kinds of properties of a compound.

In a second aspect, some embodiments of the present disclosure provide an apparatus for training a compound property prediction model. The apparatus includes: a first acquiring module, configured to acquire an unannotated compound data set; a first training module, configured to pre-train a graph neural network using the unannotated compound data set to obtain a pre-trained graph neural network; a second acquiring module, configured to acquire a plurality of annotated compound data sets, each annotated compound data set being annotated with one kind of compound property; a second training module, configured to perform multi-task training on the pre-trained graph neural network using the plurality of annotated compound data sets, to obtain a compound property prediction model, the compound property prediction model being used to predict a plurality kinds of properties of a compound.

In a third aspect, some embodiments of present disclosure provide a non-transitory computer readable storage medium, storing computer instructions, wherein the computer instructions, when executed by a computer, cause the computer to perform the method according to any one of the implementations described in the first aspect.

In a fourth aspect, some embodiments of the present disclosure provide a computer program product, the computer program product include a computer program, where the computer program, when executed by a processor, causes the processor to implement the method according to any one of the implementations described in the first aspect.

It should be understood that the content described in this part is not intended to identify key or important features of the embodiments of the present disclosure, and is not used to limit the scope of the present disclosure. Other features of the present disclosure will be easily understood through the following description.

### BRIEF DESCRIPTION OF THE DRAWINGS

After reading detailed descriptions of non-limiting embodiments given with reference to the following accompanying drawings, other features, objectives and advantages of the present disclosure will be more apparent. The accompanying drawings are used for a better understanding of the scheme, and do not constitute a limitation to the present disclosure. Here:
Fig. 1 is a flowchart of a method for training a compound property prediction model according to some embodiments of the present disclosure;
Fig. 2 is a flowchart of the method for training a compound property prediction model according to some embodiments of the present disclosure;
Fig. 3 is a flowchart of the method for training a compound property prediction model according to some embodiments of the present disclosure;
Fig. 4 is a three-stage compound pre-training and attribute prediction framework;
Fig. 5 is a schematic structural diagram of an apparatus for training a compound property prediction model according to some embodiments of the present disclosure; and
Fig. 6 is a block diagram of an electronic device used to implement the method for training a compound property prediction model according to embodiments of the present disclosure.

### DETAILED DESCRIPTION OF EMBODIMENTS

Example embodiments of the present disclosure are described below in combination with the accompanying drawings, and various details of embodiments of the present disclosure are included in the description to facilitate understanding, and should be considered as exemplary only. Accordingly, it should be recognized by one of ordinary skill in the art that various changes and modifications may be made to the embodiments described herein without departing from the scope and spirit of the present disclosure. Also, for clarity and conciseness, descriptions for well-known functions and structures are omitted in the following description.

It should be noted that embodiments in the present disclosure and the features in the embodiments may be combined with each other on a non-conflict basis. Embodiments of the present disclosure will be described below in detail with reference to the accompanying drawings.

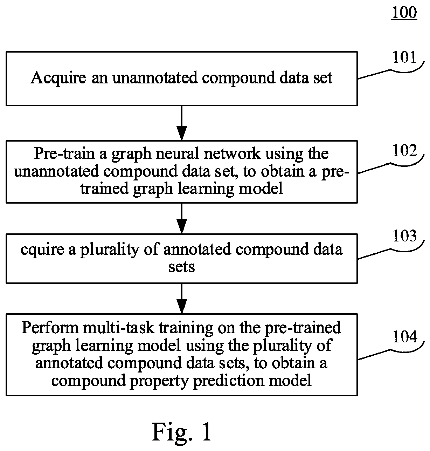

Fig. 1 illustrates a flow 100 of a method for training a compound property prediction model according to some embodiments of the present disclosure. The method for training a compound property prediction model includes the following steps.

Step 101, acquiring an unannotated compound data set.

In this embodiment, an executing body of the method for training a compound property prediction model may acquire the unannotated compound data set.

Here, the unannotated compound data set may include a large scale of compound data not annotated with a tag. The compound data may include, but not limited to, the spatial structure, topology, sequence expression, chemical knowledge, etc. of a compound.

Step 102, pre-training a graph neural network using the unannotated compound data set, to obtain a pre-trained graph neural network.

In this embodiment, the above executing body may pre-train the graph neural network (GNN) by using the unannotated compound data set, to obtain the pre-trained graph neural network.

In general, a variety of self-supervised tasks that may be applied to a compound graph structure may be designed by drawing on the pre-training technology popular in the fields of natural language processing and image processing, and the graph neural network may be pre-trained based on a large-scale unannotated compound data set, such that the model is capable of learning the physicochemical knowledge contained in the compound structure itself.

In some embodiments, the above executing body may first obtain self-supervised information based on the unannotated compound data in the unannotated compound data set; and then use the unannotated compound data as an input and the self-supervised information as an output, to pre-train the graph neural network to obtain the pre-trained graph neural network. Here, the self-supervised information may be self-supervised information mined from the compound data. A variety of self-supervised tasks applied to the compound graph structure may be designed based on the self-supervised information, and the graph neural network is pre-trained with the large-scale unannotated compound data set, such that the graph neural network is capable of learning the physicochemical knowledge contained in the compound structure itself. Here, the self-supervised information may include, but not limited to, a compound local structure (Context), a compound bond length, a compound bond angle, a molecular fingerprint, and the like. Self-supervised information is mined from a sequence expression in the compound data by using an existing chemical tool. Correspondingly, the self-supervised tasks designed based on the self-supervised information may include, but not limited to, a compound local structure prediction, a compound bond length prediction, a compound bond angle prediction, a molecular fingerprint prediction, and the like.

Step 103, acquiring a plurality of annotated compound data sets.

In this embodiment, the above executing body may acquire the plurality of annotated compound data sets.

Here, an annotated compound data set may include compound data annotated with a property tag. One annotated compound data set is annotated with one kind of compound property. Here, the plurality of annotated compound data sets may include, but not limited to, at least two of: a compound data set annotated with ADMET properties, a compound data set annotated with biological activity properties, a compound data set annotated with compound physicochemical attributes, etc.

It should be noted that the scale of the annotated compound data set is much smaller than the scale of the unannotated compound data set, and the scale of the compound data set annotated with the ADMET properties is much smaller than the scale of the compound data set annotated with the biological activity properties and the scale of the compound data set annotated with the compound physicochemical attributes.

Step 104, performing multi-task training on the pre-trained graph neural network using the plurality of annotated compound data sets, to obtain a compound property prediction model.

In this embodiment, the above executing body may perform the multi-task training on the pre-trained graph neural network using the plurality of annotated compound data sets, to obtain the compound property prediction model. Here, the compound property prediction model may be used to predict a plurality of properties of a compound.

In general, the plurality of annotated compound data sets are combined to perform the multi-task training, such that the model learns a wider range of domain knowledge, thus implementing a knowledge transfer.

In some embodiments, in addition to the compound data set annotated with the ADMET properties, other annotated compound data set of a larger scale may be collected, for example, another compound data set annotated with biologically activities, and a compound data set annotated with compound physicochemical attributes. These annotated compound data sets are combined to perform the multi-task training, such that the model learns relevance from various compound data sets annotated with properties such as physics, chemistry, biological activity and biological toxicity, thereby realizing the knowledge transfer. In particular, the effect of the model can be significantly improved by introducing the compound data set annotated with the biologically activities.

It should be noted that embodiments of the present disclosure can provide a more flexible multi-task training process. The flexible introduction and exit of a task are achieved by setting a specific number of training rounds of each task, thereby breaking the boundary between the pre-training and the fine-tuning for a downstream task. A first introduced task may be considered as pre-training in the conventional sense, and a second introduced task may be considered as downstream fine-tuning in the conventional sense. In this way, on the one hand, various customized tasks can be easily compatible, other physical, chemical and biological related data sets can be introduced, and a wider range of domain knowledge can be learned. On the other hand, by arranging the order of different tasks, the model can gradually focus on the really concerned downstream task.

According to the method for training a compound property prediction model provided by embodiments of the present disclosure, a multi-stage compound pre-training and knowledge transfer training framework is proposed. On the one hand, through the self-supervised training task, the model is pre-trained based on a large-scale unannotated compound data set, such that the model learns the physical and chemical knowledge implied in the compound structure. On the other hand, through the multi-task training, the model learns a wider range of domain knowledge, thus realizing the knowledge transfer. Thus, the effect of the model is significantly improved. Moreover, as compared with the existing supervised training, the cost of acquiring the training data is reduced. Here, the trained compound property prediction model which is capable of predicting the ADMET properties can be applied to the virtual screening in the early stage of the research and development of the drug, and thus can more accurately screen the available compound from a candidate compound library, thereby greatly reducing the risk of failure in the research and development of the drug, reducing the cost, and further greatly accelerating the discovery and development of the new drug. The large-scale compound pre-training technique is applied to the ADMET property prediction task of a compound, and the prediction accuracy of the ADMET properties is greatly improved based on the multi-stage and multi-task training framework.

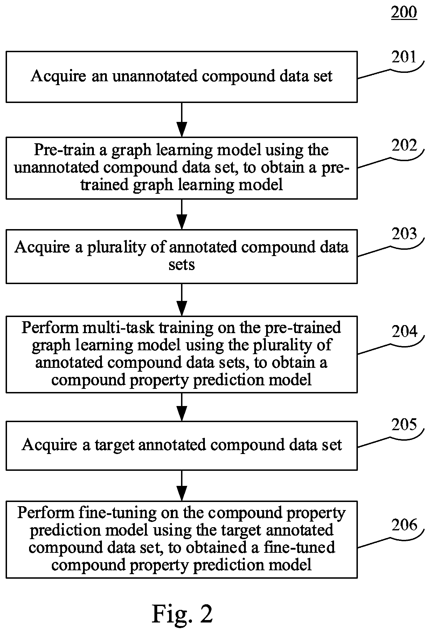

Further referring to Fig. 2, Fig. 2 illustrates a flow 200 of a method for training a compound property prediction model according to some embodiments of the present disclosure. The method for training a compound property prediction model includes the following steps.

Step 201, acquiring an unannotated compound data set.

Step 202, pre-training a graph neural network using the unannotated compound data set, to obtain a pre-trained graph neural network.

Step 203, acquiring a plurality of annotated compound data sets.

Step 204, performing multi-task training on the pre-trained graph neural network using the plurality of annotated compound data sets, to obtain a compound property prediction model.

In this embodiment, the detailed operations of step 201-204 have been described in detail in steps 101-104 in the embodiment shown in Fig. 1, and thus will not be repeatedly described here.

Step 205, acquiring a target annotated compound data set.

In this embodiment, an executing body of the method for training a compound property prediction model may acquire the target annotated compound data set.

Here, the target annotated compound data set may be annotated with a target kind of compound property. In practical applications, the target annotated compound data set may be a private data set uploaded by a user. In general, a model fine-tuning interface that can be invoked by a user may be provided, and the user may upload the private data set through the model fine-tuning interface.

Step 206, performing fine-tuning on the compound property prediction model using the target annotated compound data set, to obtain a fine-tuned compound property prediction model.

In this embodiment, the above executing body may perform the fine-tuning on the compound property prediction model using the target annotated compound data set, to obtain the fine-tuned compound property prediction model. Here, the fine-tuned compound property prediction model may be used to predict the target kind of property of a compound.

Here, based on a multi-stage compound pre-training and knowledge transfer training framework, fine-tuning task on the pre-trained model is added for the ADMET prediction task. The fine-tuning is performed on the compound property prediction model based on the private data set uploaded through the model fine-tuning interface.

It can be seen from Fig. 2 that, as compared with the embodiment corresponding to Fig. 1, a model fine-tuning step is added to the flow 200 of the method for training a compound property prediction model in this embodiment. Thus, according to the scheme described in this embodiment, a model fine tuning function is provided on the ADMET prediction task, based on the multi-stage compound pre-training and knowledge transfer training framework. The fine-tuning is performed on the compound property prediction model based on the private data set uploaded through the model fine-tuning interface, and thus, the effect of the model is significantly improved as compared with a model trained from random initialization.

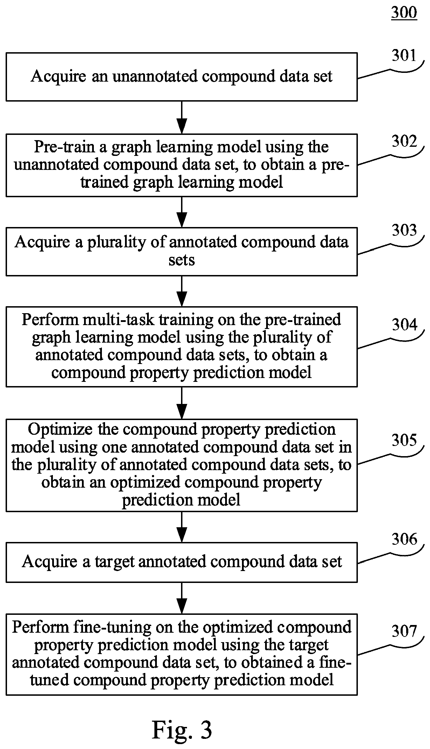

Further referring to Fig. 3, Fig. 3 illustrates a flow 300 of a method for training a compound property prediction model according to some embodiments of the present disclosure. The method for training a compound property prediction model includes the following steps.

Step 301, acquiring an unannotated compound data set.

Step 302, pre-training a graph neural network using the unannotated compound data set, to obtain a pre-trained graph neural network.

Step 303, acquiring a plurality of annotated compound data sets.

Step 304, performing multi-task training on the pre-trained graph neural network using the plurality of annotated compound data sets, to obtain a compound property prediction model.

In this embodiment, the detailed operations of step 301-304 have been described in detail in steps 101-104 in the embodiment shown in Fig. 1, and thus will not be repeatedly described here.

Step 305, optimizing the compound property prediction model using one annotated compound data set in the plurality of annotated compound data sets, to obtain an optimized compound property prediction model.

In this embodiment, the above executing body may optimize the compound property prediction model using the one annotated compound data set in the plurality of annotated compound data sets, to obtain the optimized compound property prediction model. Here, the optimized compound property prediction model may be used to predict one kind of property of a compound.

Generally, after the pre-training and the multi-task training, the shared model parameters of the compound property prediction model may be replicated and re-considered as an independent model, and the training is continued in a single-task mode for several rounds on the compound property prediction model, such that the model parameters are further optimized for a downstream task, thereby improving the effect of the model. As an example, the compound property prediction model is further trained using the compound data set annotated with the ADMET properties for several rounds, such that the optimized compound property prediction model is used to predict a property in the ADMET properties of a compound. As another example, the compound property prediction model is further trained using the compound data set annotated with the other biological activities for several rounds, such that the optimized compound property prediction model is used to predict the other biological activities of a compound.

Step 306, acquiring a target annotated compound data set.

In this embodiment, the executing body of the method for training a compound property prediction model may acquire the target annotated compound data set.

Here, the target annotated compound data set may be annotated with a target kind of compound property. In practical applications, the target annotated compound data set may be a private data set uploaded by a user. In general, a model fine-tuning interface that may be invoked by a user is provided, and the user may upload the private data set through the model fine-tuning interface.

Step 307, performing fine-tuning on the optimized compound property prediction model using the target annotated compound data set, to obtain a fine-tuned compound property prediction model.

In this embodiment, the above executing body may perform the fine-tuning on the compound property prediction model using the target annotated compound data set, to obtain the fine-tuned compound property prediction model. Here, the fine-tuned compound property prediction model may be used to predict a target kind of compound property of a compound.

Here, based on a multi-stage compound pre-training and knowledge transfer training framework, a large-scale pre-training based model fine-tuning task is added to an ADMET prediction task. The fine-tuning is performed on the compound property prediction model based on the private data set uploaded through the model fine-tuning interface.

It can be seen from Fig. 3 that, as compared with the embodiment corresponding to Fig. 1, a model optimization step and a model fine-tuning step are added to the flow 300 of the method for training a compound property prediction model in this embodiment. Thus, according to the scheme described in this embodiment, after the pre-training and the multi-task training, the training is continued in a single-task mode for several rounds, such that the model parameters are further optimized for a downstream task, thereby improving the effect of the model. Moreover, a model fine tuning function is provided on the ADMET prediction task, based on the multi-stage compound pre-training and knowledge transfer training framework. The fine-tuning is performed on the compound property prediction model based on the private data set uploaded through the model fine-tuning interface, and thus, the effect of the model is significantly improved as compared with a model trained from random initialization.

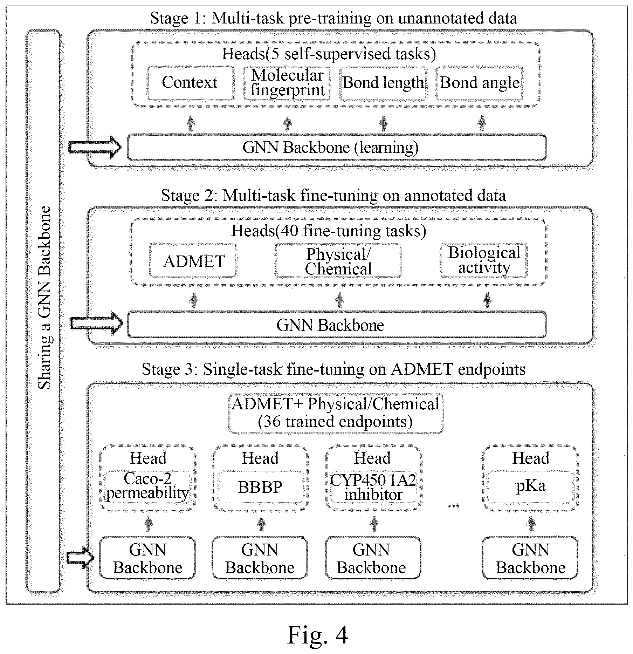

For ease of understanding, Fig. 4 shows a three-stage compound pre-training and attribute prediction framework. As shown in Fig. 4, the first stage is multi-task pre-training based on unannotated data. In the first stage, five self-supervised tasks (a compound local structure prediction, a compound molecular fingerprint prediction, a compound bond length prediction, and a compound bond angle prediction) that may be applied to the compound graph structure are designed, and the GNN backbone network is pre-trained based on a large-scale unannotated compound data set, such that the model learns the physicochemical knowledge contained in the compound structure itself. The second stage is multi-task fine-tuning on annotated data. In the second stage, a compound data set annotated with ADMET properties, a compound data set annotated with other biological activities, a compound data set annotated with compound physicochemical attributes, etc. are collected, and 40 fine-tuning tasks are constructed to perform multi-task training, such that the model learns relevance from various data sets such as physics, chemistry, biological activity and biological toxicity, thereby realizing the knowledge transfer. The third stage is single-task fine-tuning on ADMET endpoints. In the third stage, the shared model parameters are replicated and re-considered as an independent model, and 36 trained endpoints are constructed for properties such as a Caco-2 permeability, BBBP, a CYP450 1A2 inhibitor, and pKa, to continue to perform the training in a single-task mode for several rounds, such that the model parameters are further optimized for a downstream task, thereby improving the effect.

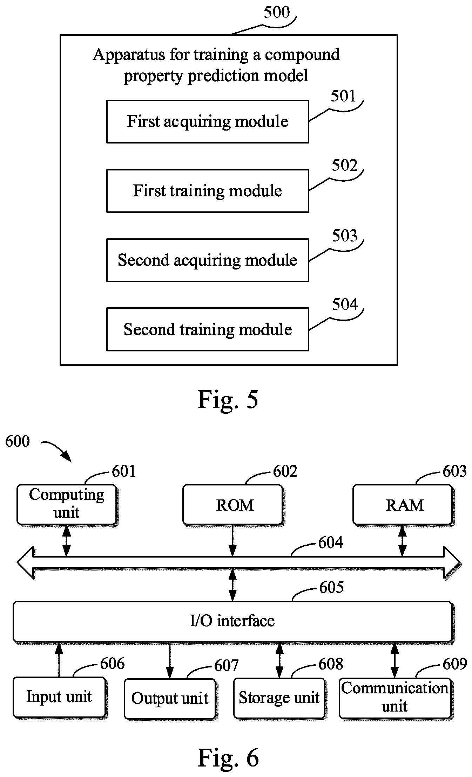

Further referring to Fig. 5, as an implementation of the method shown in the above drawing, some embodiments of the present disclosure provides an apparatus for training a compound property prediction model. The embodiments of the apparatus correspond to the embodiments of the method shown in Fig. 1. The apparatus may be applied in various electronic devices.

As shown in Fig. 5, an apparatus 500 for training a compound property prediction model in this embodiment may include: a first acquiring module 501, a first training module 502, a second acquiring module 503 and a second training module 504. Here, the first acquiring module 501 is configured to acquire an unannotated compound data set. The first training module 502 is configured to pre-train a graph neural network using the unannotated compound data set to obtain a pre-trained graph neural network. The second acquiring module 503 is configured to acquire a plurality of annotated compound data sets, each annotated compound data set being annotated with one kind of compound property. The second training module 504 is configured to perform multi-task training on the pre-trained graph neural network using the plurality of annotated compound data sets, to obtain a compound property prediction model, the compound property prediction model being used to predict a plurality kinds of properties of a compound.

In this embodiment, for detailed processes of the first acquiring module 501, the first training module 502, the second acquiring module 503 and the second training module 504 in the apparatus 500 for training a compound property prediction model, and their technical effects, reference may be respectively made to relative descriptions of steps 101-104 in the corresponding embodiment of Fig. 1, and thus, the details will not be repeatedly described here.

In some alternative implementations of this embodiment, the apparatus 500 for training a compound property prediction model further includes: a third acquiring module, configured to acquire a target annotated compound data set, the target annotated compound data set being annotated with a target kind of compound property; and a first fine-tuning module, configured to perform fine-tuning on the compound property prediction model using the target annotated compound data set, to obtain a fine-tuned compound property prediction model, the fine-tuned compound property prediction model being used to predict the target kind of compound property of a compound.

In some alternative implementations of this embodiment, the apparatus 500 for training a compound property prediction model further includes: an optimizing module, configured to optimize the compound property prediction model using one annotated compound data set in the plurality of annotated compound data sets, to obtain an optimized compound property prediction model, the optimized compound property prediction model being used to predict one kind of compound property of a compound.

In some alternative implementations of this embodiment, the apparatus 500 for training a compound property prediction model further includes: a fourth acquiring module, configured to acquire a target annotated compound data set, the target annotated compound data set being annotated with a target kind of compound property; and a second fine-tuning module, configured to perform fine-tuning on the optimized compound property prediction model using the target annotated compound data set, to obtain a fine-tuned compound property prediction model, the fine-tuned compound property prediction model being used to predict the target kind of compound property of a compound.

In some alternative implementations of this embodiment, the first training module 502 is further configured to: obtain self-supervised information based on unannotated compound data in the unannotated compound data set; and use the unannotated compound data as an input and the self-supervised information as an output, to pre-train the graph neural network to obtain the pre-trained graph neural network.

In some alternative implementations of this embodiment, the self-supervised information includes at least one of: a compound local structure, a compound bond length, a compound bond angle, or a molecular fingerprint.

In some alternative implementations of this embodiment, the plurality of annotated compound data sets include at least two of: a compound data set annotated with absorption, distribution, metabolism, excretion and toxicity (ADMET) properties of a drug, a compound data set annotated with biological activities, and a compound data set annotated with compound physicochemical attributes.

In the technical solution of the present disclosure, the collection, storage, use, processing, transmission, provision, disclosure, etc. of the personal information of a user all comply with the provisions of the relevant laws and regulations, and do not violate public order and good customs.

According to an embodiment of the present disclosure, an electronic device, a readable storage medium and a computer program product are provided.

Fig. 6 is a schematic block diagram of an example electronic device 600 that may be used to implement embodiments of the present disclosure. The electronic device is intended to represent various forms of digital computers such as a laptop computer, a desktop computer, a workstation, a personal digital assistant, a server, a blade server, a mainframe computer, and other appropriate computers. The electronic device may alternatively represent various forms of mobile apparatuses such as personal digital processing, a cellular telephone, a smart phone, a wearable device and other similar computing apparatuses. The parts shown herein, their connections and relationships, and their functions are only as examples, and not intended to limit implementations of the present disclosure as described and/or claimed herein.

As shown in Fig. 6, the device 600 includes a computation unit 601, which may perform various appropriate actions and processing, based on a computer program stored in a read-only memory (ROM) 602 or a computer program loaded from a storage unit 608 into a random access memory (RAM) 603. In the RAM 603, various programs and data required for the operation of the device 600 may also be stored. The computation unit 601, the ROM 602, and the RAM 603 are connected to each other through a bus 604. An input/output (I/O) interface 605 is also connected to the bus 604.

A plurality of parts in the device 600 are connected to the I/O interface 605, including: an input unit 606, for example, a keyboard and a mouse; an output unit 607, for example, various types of displays and speakers; the storage unit 608, for example, a disk and an optical disk; and a communication unit 609, for example, a network card, a modem, or a wireless communication transceiver. The communication unit 609 allows the device 600 to exchange information/data with other devices over a computer network such as the Internet and/or various telecommunication networks.

The computation unit 601 may be various general-purpose and/or dedicated processing components having processing and computing capabilities. Some examples of the computation unit 601 include, but are not limited to, central processing unit (CPU), graphics processing unit (GPU), various dedicated artificial intelligence (AI) computing chips, various computation units running machine learning model algorithms, digital signal processors (DSP), and any appropriate processors, controllers, microcontrollers, etc. The computation unit 601 performs the various methods and processes described above, such as a method for training a compound property prediction model. For example, in some embodiments, the method for training a compound property prediction model may be implemented as a computer software program, which is tangibly included in a machine readable medium, such as the storage unit 608. In some embodiments, part or all of the computer program may be loaded and/or installed on the device 600 via the ROM 602 and/or the communication unit 609. When the computer program is loaded into the RAM 603 and executed by the computation unit 601, one or more steps of the method for training a compound property prediction model described above maybe performed. Alternatively, in other embodiments, the computation unit 601 may be configured to perform the method for training a compound property prediction model by any other appropriate means (for example, by means of firmware).

Various embodiments of the systems and technologies described above herein may be implemented in digital electronic circuit systems, integrated circuit systems, field programmable gate arrays (FPGA), application specific integrated circuits (ASIC), application specific standard products (ASSP), system on chip (SOC), load programmable logic devices (CPLD), computer hardware, firmware, software, and/or combinations thereof. These various embodiments may be implemented in one or more computer programs that may be executed and/or interpreted on a programmable system including at least one programmable processor, which may be a dedicated or general programmable processor that may receive data and instructions from a storage system, at least one input device, and at least one output device, and transmit data and instructions to the storage system, the at least one input device, and the at least one output device.

The program code for implementing the methods of the present disclosure may be written in any combination of one or more programming languages. These program codes can be provided to the processor or controller of general computer, dedicated computer or other programmable data processing device, so that when executed by the processor or controller, the program code enables the functions / operations specified in the flow chart and/or block diagram to be implemented. The program code can be executed completely on the machine, partially on the machine, partially on the machine and partially on the remote machine as a separate software package, or completely on the remote machine or server.

In the context of the present disclosure, a machine readable medium may be a tangible medium which may contain or store a program for use by, or used in combination with, an instruction execution system, apparatus or device. The machine readable medium may be a machine readable signal medium or a machine readable storage medium. The computer readable medium may include, but is not limited to, electronic, magnetic, optical, electromagnetic, infrared, or semiconductor systems, apparatuses, or devices, or any appropriate combination of the above. A more specific example of the machine readable storage medium will include an electrical connection based on one or more pieces of wire, a portable computer disk, a hard disk, a random access memory (RAM), a read only memory (ROM), an erasable programmable read only memory (EPROM or flash memory), an optical fiber, a portable compact disk read only memory (CD-ROM), an optical storage device, a magnetic storage device, or any appropriate combination of the above.

To provide interaction with a user, the systems and technologies described herein may be implemented on a computer that is provided with: a display apparatus (e.g., a CRT (cathode ray tube) or an LCD (liquid crystal display) monitor) configured to display information to the user; and a keyboard and a pointing apparatus (e.g., a mouse or a trackball) by which the user can provide an input to the computer. Other kinds of apparatuses may also be configured to provide interaction with the user. For example, feedback provided to the user maybe any form of sensory feedback (e.g., visual feedback, auditory feedback, or tactile feedback); and an input may be received from the user in any form (including an acoustic input, a voice input, or a tactile input).

The systems and technologies described herein may be implemented in a computing system that includes a back-end component (e.g., as a data server), or a computing system that includes a middleware component (e.g., an application server), or a computing system that includes a front-end component (e.g., a user computer with a graphical user interface or a web browser through which the user can interact with an implementation of the systems and technologies described herein), or a computing system that includes any combination of such a back-end component, such a middleware component, or such a front-end component. The components of the system may be interconnected by digital data communication (e.g., a communication network) in any form or medium. Examples of the communication network include: a local area network (LAN), a wide area network (WAN), and the Internet.

The computer system may include a client and a server. The client and the server are generally remote from each other, and generally interact with each other through a communication network. The relationship between the client and the server is generated by virtue of computer programs that run on corresponding computers and have a client-server relationship with each other. The server may also be a distributed system server or a blockchain server.

It should be understood that the various forms of processes shown above may be used to reorder, add, or delete steps. For example, the steps disclosed in embodiments of the present disclosure may be executed in parallel, sequentially, or in different orders, as long as the desired results of the technical solutions mentioned in embodiments of the present disclosure can be implemented. This is not limited herein.

The above specific implementations do not constitute any limitation to the scope of protection of the present disclosure. It should be understood by those skilled in the art that various modifications, combinations, sub-combinations, and replacements may be made according to the design requirements and other factors. Any modification, equivalent replacement, improvement, and the like made within the principle of the present disclosure should be encompassed within the scope of protection of the present disclosure.

## Claims

1. A method for training a compound property prediction model, comprising:
acquiring (101, 201, 301) an unannotated compound data set;
pre-training (102, 202, 302) a graph neural network using the unannotated compound data set, to obtain a pre-trained graph neural network;
acquiring (103, 203, 303) a plurality of annotated compound data sets, each annotated compound data set being annotated with one kind of compound property; and
performing (104, 204, 304) multi-task training on the pre-trained graph neural network using the plurality of annotated compound data sets, to obtain a compound property prediction model, the compound property prediction model being used to predict a plurality kinds of properties of a compound.

2. The method according to claim 1, further comprising:
acquiring (205, 306) a target annotated compound data set, the target annotated compound data set being annotated with a target kind of compound property; and
performing (206, 307) fine-tuning on the compound property prediction model using the target annotated compound data set, to obtain a fine-tuned compound property prediction model, the fine-tuned compound property prediction model being used to predict the target kind of compound property of the compound.

3. The method according to claim 1, further comprising:
optimizing (305) the compound property prediction model using one annotated compound data set in the plurality of annotated compound data sets, to obtain an optimized compound property prediction model, the optimized compound property prediction model being used to predict one kind of compound property of a compound.

4. The method according to claim 3, further comprising:
acquiring (306) a target annotated compound data set, the target annotated compound data set being annotated with a target kind of compound property; and
performing (307) fine-tuning on the optimized compound property prediction model using the target annotated compound data set, to obtain a fine-tuned compound property prediction model, the fine-tuned compound property prediction model being used to predict the target kind of compound property of the compound.

5. The method according to any one of claims 1-4, wherein the pre-training (102, 202, 302) a graph neural network using the unannotated compound data set to obtain a pre-trained graph neural network comprises:
obtaining self-supervised information based on unannotated compound data in the unannotated compound data set; and
using the unannotated compound data as an input and the self-supervised information as an output, to pre-train the graph neural network to obtain the pre-trained graph neural network.

6. The method according to claim 5, wherein the self-supervised information comprises at least one of: a compound local structure, a compound bond length, a compound bond angle, or a molecular fingerprint.

7. The method according to any one of claims 1-6, wherein the plurality of annotated compound data sets comprise at least two of: a compound data set annotated with absorption, distribution, metabolism, excretion and toxicity (ADMET) properties, a compound data set annotated with biological activities, and a compound data set annotated with compound physicochemical attributes.

8. An apparatus for training a compound property prediction model, comprising:
a first acquiring module (501), configured to acquire (101, 201, 301) an unannotated compound data set;
a first training module (502), configured to pre-train (102, 202, 302) a graph neural network using the unannotated compound data set to obtain a pre-trained graph neural network;
a second acquiring module (503), configured to acquire (103, 203, 303) a plurality of annotated compound data sets, each annotated compound data set being annotated with one kind of compound property; and
a second training module (504), configured to perform (104, 204, 304) multi-task training on the pre-trained graph neural network using the plurality of annotated compound data sets, to obtain a compound property prediction model, the compound property prediction model being used to predict a plurality kinds of properties of a compound.

9. The apparatus according to claim 8, further comprising:
a third acquiring module, configured to acquire (205, 306) a target annotated compound data set, the target annotated compound data set being annotated with a target kind of compound property; and
a first fine-tuning module, configured to perform (206, 307) fine-tuning on the compound property prediction model using the target annotated compound data set, to obtain a fine-tuned compound property prediction model, the fine-tuned compound property prediction model being used to predict the target kind of compound property of the compound.

10. The apparatus according to claim 8, further comprising:
an optimizing module, configured to optimize (305) the compound property prediction model using one annotated compound data set in the plurality of annotated compound data sets, to obtain an optimized compound property prediction model, the optimized compound property prediction model being used to predict one kind of compound property of a compound.

11. The apparatus according to claim 10, further comprising:
a fourth acquiring module, configured to acquire (306) a target annotated compound data set, the target annotated compound data set being annotated with a target kind of compound property; and
a second fine-tuning module, configured to perform (307) fine-tuning on the optimized compound property prediction model using the target annotated compound data set, to obtain a fine-tuned compound property prediction model, the fine-tuned compound property prediction model being used to predict the target kind of compound property of the compound.

12. The apparatus according to any one of claims 8-11, wherein the first training module (501) is further configured to:
obtain self-supervised information based on unannotated compound data in the unannotated compound data set; and
use the unannotated compound data as an input and the self-supervised information as an output, to pre-train the graph neural network to obtain the pre-trained graph neural network,
preferably, the self-supervised information comprises at least one of: a compound local structure, a compound bond length, a compound bond angle, or a molecular fingerprint.

13. The apparatus according to any one of claims 8-12, wherein the plurality of annotated compound data sets comprise at least two of: a compound data set annotated with absorption, distribution, metabolism, excretion and toxicity (ADMET) properties, a compound data set annotated with biological activities, and a compound data set annotated with compound physicochemical attributes.

14. A non-transitory computer readable storage medium, storing computer instructions, wherein the computer instructions, when executed by a computer, cause the computer to perform the method according to any one of claims 1-7.

15. A computer program product, comprising a computer program, wherein the computer program, when executed by a processor, causes the processor to implement the method according to any one of claims 1-7.
